(19) Europäisches Patentamt / European Patent Office / Office européen des brevets

(11) **EP 4 438 160 A1**

(12) **DEMANDE DE BREVET EUROPEEN**

(43) Date de publication:
**02.10.2024 Bulletin 2024/40**

(51) Classification Internationale des Brevets (IPC):
**B01D 53/86** (2006.01)　　**B01D 53/88** (2006.01)
**A01C 3/02** (2006.01)　　**A61L 9/20** (2006.01)
**B65D 90/34** (2006.01)

(21) Numéro de dépôt: **23305420.4**

(22) Date de dépôt: **28.03.2023**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**KH MA MD TN**

(71) Demandeur: **Aria Technologies**
**92100 Boulogne Billancourt (FR)**

(72) Inventeurs:
• **ROMAND, Clément**
**92600 Asnières sur Seine (FR)**
• **ARNAUDIS, Jérôme**
**92400 Paris La Défense (FR)**
• **KELLY, Robert**
**92400 Paris La Défense (FR)**

(74) Mandataire: **Lavoix**
**2, place d'Estienne d'Orves**
**75441 Paris Cedex 09 (FR)**

(54) **DISPOSITIF DE COUVERTURE AVEC SYSTEME DE FILTRATION POUR UN BASSIN, BASSIN ET PROCEDE DE FILTRATION ASSOCIÉS**

(57) L'invention concerne un système de filtration (12) pour un bassin (10) contenant un liquide (20) émettant un effluent gazeux pollué (24) comprenant au moins un composé gazeux (26), ledit système de filtration (12) comprenant un dispositif de couverture (28) destiné à couvrir le bassin (10). Le dispositif de couverture (28) comprend au moins une première portion filtrante (34) comportant un matériau ayant des propriétés catalytiques. La première portion filtrante (34) est adaptée pour dégrader et transformer le composé gazeux (26) de l'effluent gazeux pollué (24) de sorte à obtenir un effluent gazeux purifié (47), le système de filtration (12) comprenant en outre des moyens de montage (30) du dispositif de couverture (28) adaptés au montage du dispositif de couverture (38) sur le bassin (10) de sorte à disposer la première portion filtrante (34) à distance du liquide (20).

**Fig.1**

**Description**

La présente invention concerne, selon un premier aspect, un système de filtration pour un bassin contenant un liquide émettant un effluent gazeux pollué comprenant au moins un composé gazeux, ledit système de filtration comprenant un dispositif de couverture destiné à couvrir le bassin.

En particulier, le bassin est par exemple un bassin de traitement ou de rétention d'une station d'épuration. Le liquide est par exemple de l'eau usée domestique, municipale, agricole ou industrielle.

Parmi les composés gazeux émis par ce type de bassin, on trouve notamment des composés odorants ou nocifs pour l'homme au-delà d'un certain seuil tels que l'ammoniac, le sulfure d'hydrogène, ou un ou plusieurs composés organiques volatils. On trouve également des gaz à effet de serre, tel que le protoxyde d'azote N2O, qui contribuent à dégrader l'environnement, et notamment l'air atmosphérique.

On connaît des systèmes de filtration dans lesquels le dispositif de couverture comprend une couverture rigide ou semi-rigide reposant sur le contour périphérique du bassin. Les effluents gazeux émis par le liquide du bassin s'accumulent et sont confinés dans l'espace de tête, encore appelé ciel gazeux, délimité entre la surface du liquide et la couverture pour empêcher qu'ils se répandent dans l'atmosphère.

Cependant, certains de ces effluents gazeux sont explosifs sous certaines conditions de température et de pression, ce qui peut poser des questions de sécurité. De plus, l'accumulation de ces effluents dans l'espace de tête est susceptible de venir perturber les réactions biochimiques se déroulant dans le bassin.

Généralement, on met alors en oeuvre des dispositifs d'extraction des effluents de l'espace de tête pour les traiter dans des équipements dédiés externes au bassin dont la mise en oeuvre est fastidieuse et onéreuse.

Un objet de l'invention est de proposer un système de filtration pour un bassin qui permette de traiter efficacement et facilement l'effluent gazeux pollué émis par le liquide présent dans le bassin.

A cet effet, l'objet de l'invention concerne un système de filtration du type précité, dans lequel le dispositif de couverture comprend au moins une première portion filtrante comportant un matériau ayant des propriétés catalytiques, ladite première portion filtrante définissant une surface inférieure destinée à être orientée vers le liquide du bassin et une surface supérieure située à l'opposé de la surface inférieure, ladite première portion filtrante étant adaptée pour dégrader et transformer le composé gazeux de l'effluent gazeux pollué de sorte à obtenir un effluent gazeux purifié, le système de filtration comprenant en outre des moyens de montage du dispositif de couverture adaptés au montage du dispositif de couverture sur le bassin de sorte à disposer la première portion filtrante à distance du liquide.

Ainsi, grâce à ses propriétés photocatalytiques, la première portion filtrante permet de traiter l'effluent gazeux en dégradant et en transformant le composé gazeux de l'effluent pollué. La première portion filtrante est disposée à distance du liquide pour augmenter l'efficacité de la réaction photocatalytique. Le système de filtration est particulièrement avantageux car il est passif et fonctionne notamment grâce au rayonnement lumineux naturel qui active la photocatalyse et la dégradation du composé gazeux adsorbé.

Selon des modes de réalisation différents, le système de filtration comprend en outre une ou plusieurs des caractéristiques suivantes, prises isolément ou selon toutes les combinaisons techniquement possibles :

- le dispositif de couverture comprend au moins une portion imperméable à l'air et à l'eau délimitant au moins une première ouverture traversante, la première portion filtrante obturant la première ouverture traversante ;

- le dispositif de couverture comprend au moins un organe de fixation amovible pour fixer de manière amovible la première portion filtrante sur la portion imperméable ;

- le composé gazeux est choisi parmi : le protoxyde d'azote, l'ammoniac, le sulfure d'hydrogène, et un composé organique volatil ;

- le système de filtration comprend en outre un dispositif lumineux comportant au moins une source lumineuse supérieure adaptée pour émettre un rayonnement lumineux orienté vers la surface supérieure de la première portion filtrante ;

- le rayonnement lumineux de la source lumineuse supérieure a une longueur d'onde au moins comprise entre 320 nm et 400 nm ;

- le dispositif lumineux comprend au moins une source lumineuse inférieure adaptée pour émettre un rayonnement lumineux orienté vers la surface inférieure de la première portion filtrante, le rayonnement lumineux de la source lumineuse inférieure ayant de préférence une longueur d'onde au moins comprise entre 320 nm et 400 nm ;

- le dispositif lumineux comprend une unité de contrôle configurée pour contrôler une mise en marche ou un arrêt de la source lumineuse supérieure, et préférentiellement de la source lumineuse inférieure, en fonction d'un horaire et/ou d'une date, ou en fonction d'une concentration en composé gazeux ;

- le système de filtration comprend en outre un dispositif de protection des intempéries comportant une membrane étanche à l'eau, perméable à l'air et translucide disposée sur la surface supérieure de la première portion filtrante, ou comportant un matériau translucide disposé à distance de la surface supérieure de la première portion filtrante ;

- le matériau ayant des propriétés catalytiques comprend une couche adsorbante adaptée pour adsorber le composé gazeux, et une couche photocatalytique comprenant au moins un élément photocatalytique adapté pour dégrader et transformer le composé gazeux adsorbé sur la couche adsorbante ; et

- l'élément photocatalytique est l'oxyde de titane sous forme rutile.

L'invention concerne aussi, selon un deuxième aspect, un bassin contenant un liquide émettant un effluent gazeux pollué, l'effluent gazeux pollué comprenant au moins un composé gazeux, ledit bassin comprenant un système filtration tel que décrit plus haut, le dispositif de couverture étant monté sur le bassin, la surface inférieure de la première portion filtrante étant orientée vers le liquide du bassin, le dispositif de couverture, la surface du liquide et au moins partie d'une paroi du bassin délimitant entre eux un espace de tête contenant l'effluent gazeux pollué.

Selon un mode de réalisation particulier, au moins une partie de la première portion filtrante s'étend dans un plan formant un angle non nul avec la surface du liquide.

Selon un troisième aspect, l'invention concerne un procédé de filtration d'un effluent gazeux pollué émis par un liquide contenu dans un bassin tel que décrit plus haut, ledit procédé comprenant les étapes suivantes :

montage du dispositif de couverture sur le bassin avec les moyens de montage de sorte à disposer la première portion filtrante à distance du liquide,
filtration de l'effluent gazeux pollué par le passage dudit effluent au travers de la première portion filtrante de sorte à obtenir un effluent gazeux purifié.

Selon un mode de réalisation particulier, le procédé comprend en outre une étape de contrôle d'une efficacité de filtration de la première portion filtrante, ladite étape comprenant les sous-étapes suivantes : mesure d'une première concentration en composé gazeux de l'effluent gazeux pollué dans l'espace de tête, mesure d'une deuxième concentration en composé gazeux dans l'effluent gazeux purifié, calcul d'un paramètre représentatif de l'efficacité de filtration en fonction de la première concentration et de la deuxième concentration, remplacement ou nettoyage de la première portion filtrante en fonction du paramètre représentatif de l'efficacité de filtration.

D'autres aspects et avantages de l'invention apparaîtront à la lecture de la description qui suit, donnée à titre d'exemple et faite en référence aux dessins annexés, parmi lesquels :

- la figure 1 est une coupe schématique d'un bassin comprenant un système de filtration selon un premier mode de réalisation de l'invention,
- la figure 2 est une vue du dessus du bassin de la figure 1,
- la figure 3 est une représentation en coupe du matériau de la première portion filtrante du dispositif de couverture du système de filtration du bassin de la figure 1,
- les figures 4 à 5 sont des représentations schématiques, respectivement en coupe, vue du dessus et en perspective d'un système de filtration selon un deuxième mode de réalisation de l'invention,
- la figure 6 est une représentation schématique d'un système de filtration selon un troisième mode de réalisation de l'invention.

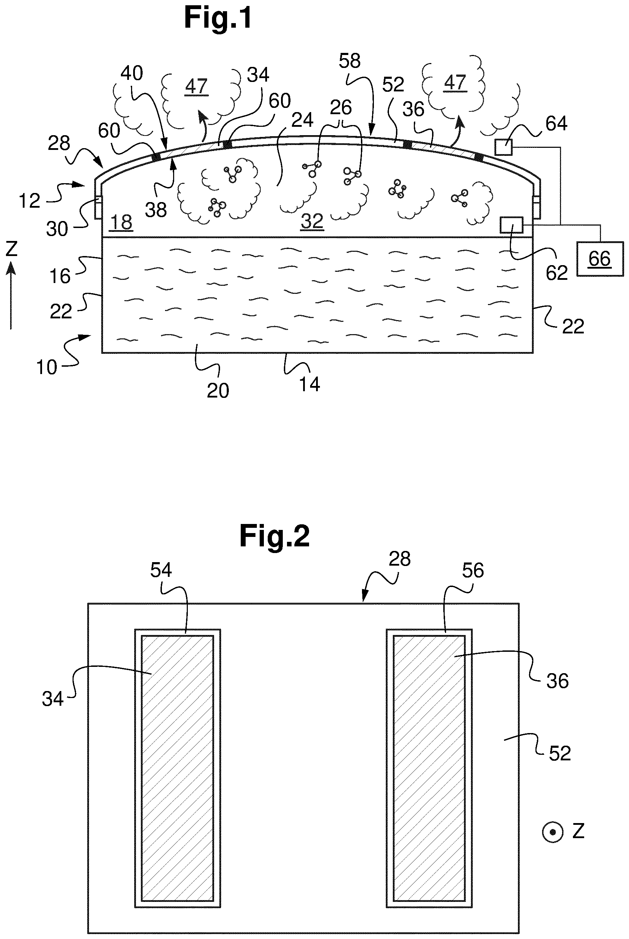

Sur les figures 1 et 2, on a représenté, de manière schématique, respectivement en coupe et en vue du dessus, un bassin 10 selon un premier mode de réalisation.

Le bassin 10 comprend un système de filtration 12 selon un premier mode de réalisation de l'invention.

Le bassin 10 est destiné à être disposé sur un sol. Le bassin 10 comprend une paroi de fond 14 et une paroi latérale 16 reliée à la paroi de fond 14. La paroi latérale 16 et la paroi de fond 14 délimitent entre elles un volume intérieur 18 recevant un liquide 20. Par exemple, comme représenté sur la figure 2, la paroi de fond 14 a une forme rectangulaire et la paroi latérale 16 comprend quatre parois rectangulaires 22 reliées entre elles et à la paroi de fond 14. En variante, la paroi de fond 14 est circulaire et la paroi latérale 16 est une paroi cylindrique de révolution à base circulaire. Il est entendu que la paroi de fond 14 et la paroi latérale 16 du bassin 10 peuvent avoir une forme quelconque.

Le bassin 10 est par exemple un bassin de traitement ou de rétention d'une station d'épuration.

Le liquide 20 est un liquide émettant un effluent gazeux pollué 24. Par « pollué », on entend que l'effluent gazeux 24 comprend au moins un composé gazeux 26 désagréable, odorant ou nocif pour l'homme au-delà d'un certain seuil tels que l'ammoniac, le sulfure d'hydrogène, ou un ou plusieurs composés organiques volatils. En variante, le composé gazeux est un gaz à effet de serre, tels que le protoxyde d'azote $N2O$, qui contribue à dégrader l'environnement, et notamment l'air atmosphérique. La concentration en composé gazeux 26 est par exemple comprise entre 0,001 pm et 100 ppm dans l'effluent gazeux pollué 24.

Le liquide 20 est par exemple de l'eau usée domestique, municipale, agricole et/ou industrielle.

Le bassin 10 comprend par exemple une ou plusieurs conduites d'alimentation (non représentées) destinées à amener le liquide 20 à l'intérieur du volume intérieur 18 du bassin 10. Le bassin 10 comprend en outre, de préférence, une ou plusieurs conduites d'extraction (non représentées) destinées à extraire le liquide 20 du bassin 10.

Selon l'invention, le système de filtration 12 comprend un dispositif de couverture 28 et des moyens de montage 30 du dispositif de couverture 28 adaptés au montage du dispositif de couverture 28 sur le bassin 10, en particulier sur le périmètre du bassin 10.

Le dispositif de couverture 28, la surface du liquide 20 contenu dans le bassin 10 et une paroi 16 du bassin 10, en particulier la paroi latérale 16, délimitent entre eux un espace de tête 32 contenant l'effluent gazeux pollué 24.

Le dispositif de couverture 28 comprend au moins une première portion filtrante 34 comportant un matériau 35 ayant des propriétés photocatalytiques.

Dans l'exemple des figures 1 et 2, le dispositif de couverture 28 comprend en outre une deuxième portion filtrante 36 comportant un matériau 35 ayant des propriétés catalytiques. De préférence, la première portion filtrante 34 et la deuxième portion filtrante 36 sont faites du même matériau 35 ayant des propriétés photocatalytiques. Chaque première et deuxième portions filtrantes 34, 36 comprend une surface inférieure 38 orientée vers la surface du liquide 20 et une surface supérieure 40, à l'opposé de la surface inférieure 38 selon une direction d'élévation Z.

Il est entendu que, selon l'invention, le dispositif de couverture 28 peut comprendre un nombre quelconque de portions filtrantes 34, 36.

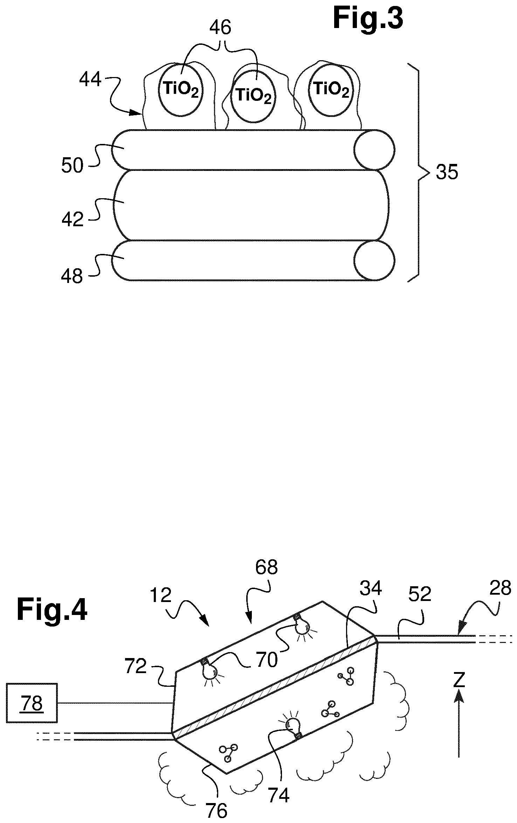

Avantageusement, comme représenté sur la figure 3, le matériau 35 ayant des propriétés photocatalytiques comprend une couche adsorbante 42 adaptée pour adsorber le composé gazeux 26 et une couche photocatalytique 44 comprenant au moins un élément photocatalytique 46 adaptée pour dégrader et transformer le composé gazeux 26 adsorbé sur la couche adsorbante 42, de sorte à obtenir un effluent gazeux purifié 47 en aval de la première portion filtrante 34 et/ou de la deuxième portion filtrante 36.

Par « effluent gazeux purifié », on entend que la concentration en composé gazeux dans ledit effluent gazeux purifié 47 est inférieure à la concentration en composé gazeux 26 dans l'effluent gazeux pollué situé dans l'espace de tête 32 en amont de la première portion filtrante 34.

La couche adsorbante 42 est orientée du côté du liquide 20. La couche photocatalytique 44 est orientée à l'opposé de la couche adsorbante 42.

De préférence, le matériau 35 comprend en outre une première couche de support 48 perméable à l'air fixée sur une surface inférieure de la couche adsorbante 42, et une deuxième couche de support 50 perméable à l'air fixée sur la surface supérieure de la couche adsorbante 42, entre la couche adsorbante 42 et la couche photocatalytique 44.

La couche adsorbante 42 est par exemple une couche à base de charbon actif. En variante, la couche adsorbante 42 est une couche à base de fibres non tissées de noix de coco ou de bois. La nature du matériau de la couche adsorbante 42 est avantageusement choisie en fonction de ses qualités d'adsorption et selon la charge d'effluent gazeux pollué 24 émise par le liquide 20 du bassin 10.

La première couche de support 48 et la deuxième couche de support 50 sont de préférence de même nature et sont, par exemple, des non tissés à base de fibres naturelles et/ou de fibres chimiques organiques.

L'élément photocatalytique 46, encore appelé agent photocatalyseur, est de préférence un solide semi-conducteur, par exemple le dioxyde de titane ($TiO_2$). En variante, l'élément photocatalytique 46 est un oxyde alcalino-terreux, un oxyde d'actinide ou un oxyde de terre rare.

La photocatalyse est initiée en activant l'élément photocatalytique 46 par des rayonnements ultraviolets provoquant des changements électroniques au sein de l'élément photocatalytique 46 et conduisant en présence du composé gazeux 26, à la création de radicaux oxygénés ou hydroxyles à la surface de l'élément photocatalytique 46. Ces radicaux attaquent le composé gazeux 26 adsorbé et par succession de réactions électrochimiques dégradent le composé gazeux 26 jusqu'au stade final de l'oxydation.

Avantageusement, l'élément photocatalytique 46 est le dioxyde de titane sous forme rutile. Ceci est particulièrement avantageux car il confère à la portion filtrante 34, 36 des propriétés hydrophobes qui permettent d'éviter la saturation en eau de la portion filtrante 34, 36 due aux intempéries, et ainsi d'augmenter l'efficacité de la réaction photocatalytique.

Par exemple, la première portion filtrante 34 et la deuxième portion filtrante 36 sont faites d'un matériau tels que ceux décrits dans WO 0213950 A1.

Avantageusement, la masse volumique et l'épaisseur de la première portion filtrante 34 et de la deuxième portion filtrante 36 sont choisies par l'homme du métier pour que le dispositif de couverture 28 induise une faible perte de charge, par exemple entre 10 Pa et 500 Pa. Ceci permet de ne pas perturber les réactions biochimiques qui se déroulent à l'intérieur du bassin 10.

Par exemple, les masses volumiques de la première portion filtrante 34 et de la deuxième portion filtrante 36 sont comprises entre 100 g/m$^2$ et 1000 g/m$^2$

Par exemple, les épaisseurs de la première portion filtrante 34 et de la deuxième portion filtrante 36 sont comprises entre 1 mm et 50 mm.

De préférence, comme illustré sur les figures 1 et 2, le dispositif de couverture 28 comprend au moins une portion imperméable 52 à l'air et à l'eau. La portion imperméable 52 délimite au moins une première ouverture traversante 54. La première portion filtrante 34 obture la première ouverture traversante 54. Par « obturer », on entend que la première portion filtrante 34 ferme entièrement la première ouverture traversante 54 obligeant l'effluent gazeux pollué 24 à traverser la première portion filtrante 34.

Dans l'exemple des figures 1 et 2, la portion imperméable 52 délimite en outre une deuxième ouverture traversante 56. La deuxième portion filtrante 36 obture la deuxième ouverture traversante 56.

De préférence, la première portion filtrante 34 et la deuxième portion filtrante 36 sont maintenues tendues respectivement à l'intérieur de la première ouverture traversante 54 et de la deuxième ouverture traversante 56.

Préférentiellement, le ratio entre la surface de la portion filtrante 34 ou des portions filtrantes 34, 36 et la surface de la portion imperméable 52 est compris entre 5% et 80%, typiquement entre 15% et 60%, par exemple 50%. Le ratio est choisi par l'homme du métier en fonction de la charge de polluant, du type de liquide 20 présent dans le bassin 10, etc. pour limiter la perte de charge.

Avantageusement, au moins une partie de la première portion filtrante 34 s'étend localement dans un plan formant un angle non nul avec la surface du liquide. Ceci empêche l'accumulation de débris sur la première portion filtrante 34 et facilite le ruissellement de l'eau de pluie sur la première portion filtrante 34.

De préférence, l'angle est compris entre 30° et 60°, par exemple 45°. L'angle est par exemple choisi en fonction de la latitude de l'endroit dans lequel sont installés le bassin 10 et le système de filtration 12. De préférence encore, la première portion filtrante 34 et/ou la deuxième portion filtrante 36 sont orientées vers le sud. Ceci de permet d'exposer favorablement les portion filtrantes 34, 36 au rayonnement solaire.

Dans l'exemple représenté sur la figure 1, le dispositif de couverture 28 a une surface extérieure 58 en contact avec l'air ambiant de forme convexe. Ceci facilite également le ruissellement de l'eau de pluie. Chaque portion filtrante 34, 36 a alors une surface extérieure incurvée formant une portion de la surface extérieure 58 convexe du dispositif de couverture 28.

La portion imperméable 52 est par exemple rigide, c'est-à-dire que la portion imperméable 52 ne se déforme pas macroscopiquement par exemple lors de son installation sur le bassin. En variante, la portion imperméable 52 est flexible.

Par exemple, la portion imperméable 52 est faite d'une tôle en aluminium ou de polychlorure de vinyle. Le polychlorure de vinyle est de préférence résistant au rayonnement ultra-violet.

Avantageusement, le dispositif de couverture 28 comprend au moins un organe de fixation amovible 60 pour fixer de manière amovible chaque portion filtrante 34, 36 sur la portion imperméable 52.

Par « fixer de manière amovible », on entend que la portion filtrante 34, 36 peut être solidariser à la portion imperméable 52 et désolidariser de la portion imperméable 52 sans que l'intégrité physique de la portion filtrante 34, 36 et l'intégrité physique de la portion imperméable 52 ne soient affectées.

Par exemple, l'organe de fixation amovible 60 est une bande auto-agrippante.

En variante (non représenté), l'organe de fixation amovible 60 comprend un cadre supérieur et un cadre inférieur enserrant chacune des portions filtrantes 34, 36. L'un du cadre supérieur et du cadre inférieur est fixé sur la portion imperméable 52.

Avantageusement, la première portion filtrante 34 et la deuxième portion filtrante 36 sont fixées de manière étanche sur la portion imperméable 52, c'est-à-dire que l'organe de fixation amovible 60 empêche le passage de l'effluent gazeux pollué 24 à la jonction entre la première portion filtrante 34 et la portion imperméable 52, et à la jonction entre la deuxième portion filtrante 36 et la portion imperméable 52.

Ainsi, pour quitter l'espace de tête 32, l'effluent gazeux pollué 24 passe uniquement au travers de la première portion filtrante 34 et de la deuxième portion filtrante 36.

Les moyens de montage 30 du dispositif de couverture 28 sont adaptés au montage du dispositif de couverture 28 sur le bassin 10 de sorte à disposer la première portion filtrante 34, et le cas échéant la deuxième portion filtrante 36 dans l'exemple de la figure 1, à distance du liquide 10 contenu dans le bassin 10. Dit autrement, la première portion filtrante 34, et éventuellement la deuxième portion filtrante 36, sont situées à l'écart de la surface du liquide 20, hors du liquide 20.

Les moyens de montage 30 comprennent par exemple des rivets ou des boulons, une ou plusieurs sangles élastiques ou non. Les moyens de montage 30 sont connus de l'homme du métier.

De préférence, les moyens de montage 30 permettent de monter le dispositif de couverture 28 de manière étanche sur le bassin 10 de sorte que l'effluent gazeux pollué 24 ne puisse pas s'échapper vers l'extérieur du volume intérieur 18 au niveau de la jonction entre le bassin 10 et le dispositif de couverture 28.

Avantageusement, le système de filtration 12 comprend au moins un organe de régulation de la pression (non représenté) de l'effluent gazeux pollué 24 à l'intérieur de l'espace de tête 32. L'organe de régulation est par exemple un organe de surpression, par exemple une soupape, adapté pour mettre en communication fluidiquement l'intérieur de l'espace de tête 32 avec l'extérieur du volume intérieur 18 lorsque la pression à l'intérieur de l'espace de tête 32 est supérieure à un seuil prédéterminé, par exemple compris entre 10 et 100 Pa. Cet organe de régulation permet de ne pas modifier l'équilibre des pressions partielles du processus du bassin.

En variante ou en complément, le système de filtration 12 comprend au moins un ventilateur d'insufflation (non représenté) destiné à insuffler de l'air extérieur à l'intérieur de l'espace de tête 32 afin de diluer la concentration en composé gazeux 26 à l'intérieur de l'espace de tête 32 et optimiser la concentration en composé gazeux 26, le débit de l'effluent gazeux pollué 24 à travers le dispositif de couverture 28, et/ou le taux d'humidité de l'effluent gazeux pollué 24 pour favoriser la filtration.

Le système de filtration 12 peut en outre optionnellement comprendre un ou plusieurs organes d'homogénéisation de l'effluent gazeux pollué 24. L'organe d'homogénéisation est par exemple un ventilateur situé entre le dispositif de couverture 28 et la surface du liquide présent dans le bassin 10.

Selon un mode de réalisation particulier, le système de filtration 12 comprend au moins un premier capteur 62 configuré pour mesurer une première concentration en composé gazeux 26 dans l'espace de tête 32, c'est-à-dire dans l'effluent gazeux pollué 24, et au moins un deuxième capteur 64 configuré pour mesurer une deuxième concentration en composé gazeux 26 dans l'effluent gazeux purifié 47.

Le système de filtration 12 comprend avantageusement une unité de détermination 66 de l'efficacité de filtration du système de filtration 12 connectée au premier capteur 62 et au deuxième capteur 64. L'unité de détermination 66 est configurée pour déterminer une efficacité de filtration (E) en fonction de la première concentration (C1) et de la deuxième concentration (C2) mesurées.

Par exemple, l'efficacité de filtration (E) est calculée en utilisant l'équation :

$$E = \left(\frac{(\beta - 1)}{\beta}\right) \times 100 \text{ avec } \beta = \frac{c_1}{c_2}$$

La détermination de l'efficacité est par exemple utilisée pour décider du remplacement ou du nettoyage de la première portion filtrante 34 et/ou de la deuxième portion filtrante 36, notamment lorsque l'efficacité est inférieure à un seuil prédéterminé, par exemple 50%.

Préférentiellement, le système de filtration 12 comprend au moins une passerelle d'accès (non représentée) permettant à un opérateur d'accéder à au moins la première portion filtrante 34 pour les opérations de remplacement et/ou de nettoyage.

Un procédé de filtration d'un effluent gazeux pollué 24 selon l'invention va maintenant être décrit.

Le procédé comprend tout d'abord le remplissage du volume intérieur 18 du bassin 10 avec un liquide 20 émettant un effluent gazeux pollué 24.

On monte ensuite le dispositif de couverture 28 sur le bassin 10 avec les moyens de montage 30 de sorte à disposer chaque portion filtrante 34, 36 à distance du liquide 20.

L'effluent gazeux pollué 24 émis par le liquide 20 est ensuite filtré en passant au travers de la première portion filtrante 34 et au travers de la deuxième portion filtrante 36 de sorte à obtenir de l'autre côté du dispositif de couverture 28, c'est-à-dire dans l'atmosphère entourant le bassin 10, un effluent gazeux purifié 47.

Avantageusement, le procédé comprend en outre une étape de contrôle de l'efficacité de filtration de la première portion filtrante 34 et/ou de la deuxième portion filtrante 36.

Cette étape comprend une première sous-étape de mesure de la première concentration en composé gazeux 26 de l'effluent gazeux pollué 24 dans l'espace de tête 32 en utilisant le premier capteur 62 de concentration en composé gazeux 26 et une deuxième sous-étape de mesure de la deuxième concentration en composé gazeux 26 dans l'effluent gazeux purifié 47, en aval du dispositif de couverture 28 en utilisant le deuxième capteur 64 de concentration en composé gazeux 26.

Le procédé comprend alors une étape de calcul d'un paramètre représentatif de l'efficacité de filtration du dispositif de couverture 28 en fonction de la première concentration et de la deuxième concentration. Par exemple, le paramètre représentatif est calculé tel que décrit plus haut.

L'opérateur peut ensuite procéder au remplacement ou au nettoyage de la première portion filtrante 34 et/ou de la deuxième portion filtrante 36 en fonction du paramètre représentatif de l'efficacité de filtration, par exemple si l'efficacité E calculée précédemment est inférieure à un seuil prédéterminé.

Le nettoyage s'effectue par exemple à la vapeur d'eau et peut être suivi par un séchage.

Les figures 4 à 5 représentent un système de filtration 12 selon un deuxième mode de réalisation de l'invention.

Dans ce mode de réalisation, le système de filtration 12 comprend en outre un dispositif lumineux 68 comportant au moins une source lumineuse supérieure 70 adaptée pour émettre un rayonnement lumineux orienté vers la surface supérieure de la première portion filtrante 34.

De préférence, comme illustré, le dispositif lumineux 68 comprend une structure supérieure de support 72 maintenant la ou les sources lumineuses supérieures 70. La structure supérieure de support 72 est située au-dessus de la surface supérieure de la première portion filtrante 34 selon la direction d'élévation Z.

Le dispositif lumineux 68 est particulièrement avantageux car il permet l'activation par photocatalyse du matériau 35 présentant des propriétés photocatalytiques et en particulier de la couche photocatalytique 44.

Dans l'exemple illustré, le dispositif lumineux 68 comprend deux sources lumineuses supérieures 70 adaptées pour émettre chacune un rayonnement lumineux orienté vers la surface supérieure de la première portion filtrante 34.

Avantageusement, le dispositif lumineux 68 comprend au moins une source lumineuse inférieure 74 adaptée pour émettre un rayonnement lumineux orienté vers la surface inférieure de la première portion filtrante 34.

Pour ce faire, préférentiellement, le dispositif lumineux 68 comprend une structure inférieure de support 76 maintenant la ou les sources lumineuses inférieures 74. La structure inférieure de support 76 est située en-dessous de la surface inférieure de la première portion filtrante 34 selon la direction d'élévation Z.

Le rayonnement lumineux de la ou des sources lumineuses supérieures 70 et/ou de la ou des sources lumineuses inférieures 74 a une longueur d'onde au moins comprise entre 320 nm et 400 nm, c'est-à-dire une longueur d'onde comprise dans le rayonnement ultraviolet A pour activer la photocatalyse (UVA). Chacune des sources lumineuses supérieures 70 et/ou inférieures 74 peut bien entendu avoir un spectre de longueurs d'ondes plus étendu, par exemple un spectre similaire à celui de la lumière naturelle, c'est-à-dire compris entre 200 nm et 2000 nm.

Avantageusement, la source lumineuse supérieure 70 et/ou la source lumineuse inférieure 74 est une source lumineuse électroluminescente (LED), ou un tube fluorescent, ou une source lumineuse au xénon/mercure.

Selon un mode de réalisation avantageux, le dispositif lumineux 68 comprend une pluralité de sources lumineuses supérieures 70 et/ou une pluralité de sources lumineuses inférieures 74. De préférence, la pluralité de sources lumineuses supérieures 70 et/ou la pluralité de sources lumineuses inférieures 74 comprennent chacune au moins deux sources lumineuses ayant des spectres de longueurs d'onde distincts. Par exemple, la pluralité de sources lumineuses supérieures 70 et/ou la pluralité de sources lumineuses inférieures 74 comprennent chacune une source lumineuse électroluminescente (LED), un tube fluorescent, et une source lumineuse au xénon/mercure.

De préférence, la puissance lumineuse de chacune des sources lumineuses supérieures 70 et/ou inférieures 74 est choisie en fonction de la charge de polluant à traiter, c'est-à-dire en fonction de la concentration en composé gazeux 26 dans l'effluent gazeux pollué 24. La puissance lumineuse, exprimée en irradiance, est de préférence comprise entre 1 et 1000 $\mu$W/cm$^2$/nm.

Avantageusement, le dispositif lumineux 68 comprend une unité de contrôle 78 configurée pour contrôler une mise en marche ou un arrêt de la source lumineuse supérieure 70, et préférentiellement de la source lumineuse inférieure 74, en fonction d'un horaire et/ou d'une date, ou en fonction d'une concentration en composé gazeux 26.

Par exemple, l'unité de contrôle 78 est configurée pour mettre en marche la source lumineuse supérieure 70 et/ou la source lumineuse inférieure 74 durant la nuit et arrêter la source lumineuse supérieure 70 et/ou la source lumineuse inférieure 74 durant le jour. Le contrôle s'effectue par exemple sur la base d'horaires prédéterminés (par exemple le lever et le coucher du soleil) ou sur la base d'une luminosité mesurée par un capteur de luminosité (non représenté).

En variante ou en complément, la mise en marche ou l'arrêt de la source lumineuse supérieure 70 et/ou de la source lumineuse inférieure 74 sont contrôlés en fonction de la première concentration en composé gazeux 26 mesuré dans l'espace de tête 32, de préférence en utilisant le premier capteur 62 de concentration. L'unité de contrôle 78 est alors configurée pour mettre en marche la source lumineuse supérieure 70 et/ou la source lumineuse inférieure 74 lorsque la première concentration dépasse un seuil prédéterminé.

Préférentiellement, le procédé comprend une étape de contrôle de la mise en marche ou de l'arrêt de la source lumineuse supérieure 70, et préférentiellement de la source lumineuse inférieure 74, en fonction d'un horaire et/ou d'une date, ou en fonction d'une concentration en composé gazeux 26.

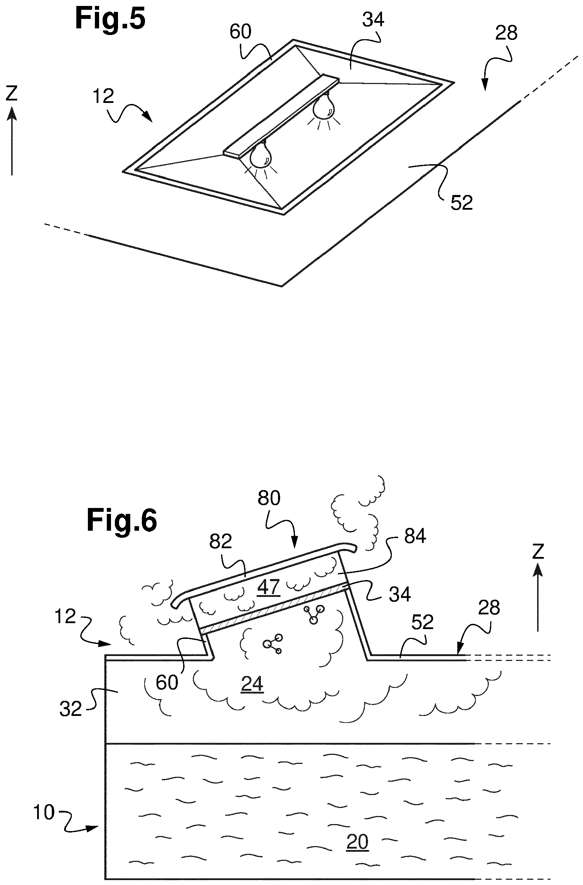

La figure 6 présente un système de filtration 12 selon un troisième mode de réalisation. Ce mode de réalisation sera décrit par différences par rapport au deuxième mode de réalisation.

Dans ce mode de réalisation, le système de filtration 12 comprend un dispositif de protection des intempéries 80 destiné à protéger la première portion filtrante 34 et, préférentiellement la deuxième portion filtrante 36 des intempéries.

Dans l'exemple illustré, le dispositif de protection des intempéries 80 comprend un matériau translucide 82 disposé à distance de la surface supérieure de la première portion filtrante 34 et préférentiellement à distance de la deuxième portion filtrante 36. Le matériau 82 couvre et protège au moins la première portion filtrante 34 et/ou la deuxième portion filtrante 36. En variante, le matériau 82 couvre en outre au moins une partie de la portion imperméable 52. Le dispositif de protection des intempéries 80 est par exemple une plaque.

Par « translucide », on entend un matériau 82 avec un taux de transmission de la lumière compris entre 5% et 100%. La lumière comprend au moins des ondes électromagnétiques dont la longueur d'onde est comprise entre 320 nm et 400 nm. Dit autrement, le matériau 82 laisse passer au moins une partie du spectre de la lumière dans le domaine des UVA de sorte à permettre la réaction de photocatalyse.

Le matériau 82 est par exemple du poly méthacrylate de méthyle acrylique encore appelé plexiglas. En variante, le matériau 82 est du verre de quartz.

Le dispositif de protection des intempéries 80 et la surface supérieure du dispositif de couverture 28, et en particulier la surface supérieure de la première portion filtrante 34 et/ou de la deuxième portion filtrante 36 délimitent entre elles un canal de circulation d'air 84 qui permet à l'effluent gazeux purifié 47 de circuler et de s'échapper dans l'atmosphère qui entoure le système de filtration 12.

En variante (non représentée), le dispositif de protection des intempéries 80 comprend une membrane étanche à l'eau et perméable à l'air pour permettre le passage de l'effluent gazeux purifié 47, et translucide. La membrane est alors disposée sur la surface supérieure d'au moins la première portion filtrante 34 au contact avec la première portion filtrante 34.

Le dispositif de protection des intempéries 80 permet d'empêcher la première portion filtrante 34 d'être saturée en eau empêchant la réaction de photocatalyse.

Bien entendu, selon un mode de réalisation particulier, le dispositif de protection des intempéries 80 peut former la structure supérieure de support 72 du dispositif lumineux 68.

Ainsi, le système de filtration 12 selon l'invention est particulièrement avantageux car, la première portion filtrante 34, grâce à ses propriétés photocatalytiques, permet de traiter l'effluent gazeux pollué 24 en dégradant et en transformant le composé gazeux 26 de l'effluent gazeux pollué 24. La première portion filtrante 34 est disposée à distance du liquide 20 pour augmenter l'efficacité de la réaction photocatalytique. Le système de filtration 12 est particulièrement avantageux car il est passif et fonctionne en particulier grâce au rayonnement lumineux naturel qui active la photocatalyse et la dégradation du composé gazeux 26 adsorbé.

## Revendications

1. Système de filtration (12) pour un bassin (10) contenant un liquide (20) émettant un effluent gazeux pollué (24) comprenant au moins un composé gazeux (26), ledit système de filtration (12) comprenant un dispositif de couverture (28) destiné à couvrir le bassin (10), **caractérisé en ce que** ledit dispositif de couverture (28) comprend au moins une première portion filtrante (34) comportant un matériau (35) ayant des propriétés catalytiques, ladite première portion filtrante (34) définissant une surface inférieure (38) destinée à être orientée vers le liquide (20) du bassin (10) et une surface supérieure (40) située à l'opposé de la surface inférieure (38), ladite première portion filtrante (34) étant adaptée pour dégrader et transformer le composé gazeux (26) de l'effluent gazeux pollué (24) de sorte à obtenir un effluent gazeux purifié (47), le système de filtration (12) comprenant en outre des moyens de montage (30) du dispositif de couverture (28) adaptés au montage du dispositif de couverture (38) sur le bassin (10) de sorte à disposer la première portion filtrante (34) à distance du liquide (20).

2. Système de filtration (12) selon la revendication 1, dans lequel le dispositif de couverture (28) comprend au moins une portion imperméable (52) à l'air et à l'eau délimitant au moins une première ouverture traversante (54), la première portion filtrante (34) obturant la première ouverture traversante (54).

3. Système de filtration (12) selon la revendication 2, dans lequel le dispositif de couverture (28) comprend au moins un organe de fixation amovible (60) pour fixer de manière amovible la première portion filtrante (34) sur la portion imperméable (52).

4. Système de filtration (12) selon l'une quelconque des revendications 1 à 3, dans lequel le composé gazeux (26) est choisi parmi : le protoxyde d'azote, l'ammoniac, le sulfure d'hydrogène, et un composé organique volatil.

5. Système de filtration (12) selon l'une quelconque des revendications 1 à 4, dans lequel le système de filtration (12) comprend en outre un dispositif lumineux (68) comportant au moins une source lumineuse supérieure (70) adaptée pour émettre un rayonnement lumineux orienté vers la surface supérieure (40) de la première portion filtrante (34).

6. Système de filtration (12) selon la revendication 5, dans lequel le rayonnement lumineux de la source lumineuse supérieure (70) a une longueur d'onde au moins comprise entre 320 nm et 400 nm.

7. Système de filtration (12) selon l'une quelconque des revendications 1 à 6, dans lequel le dispositif lumineux (68) comprend au moins une source lumineuse inférieure (74) adaptée pour émettre un rayonnement lumineux orienté vers la surface inférieure (38) de la première portion filtrante (34), le rayonnement lumineux de la source lumineuse inférieure (74) ayant de préférence une longueur d'onde au moins comprise entre 320 nm et 400 nm.

8. Système de filtration (12) selon l'une quelconque des revendications 5 à 7, dans lequel le dispositif lumineux (68) comprend une unité de contrôle (78) configurée pour contrôler une mise en marche ou un arrêt de la source lumineuse supérieure (70), et préférentiellement de la source lumineuse inférieure (74), en fonction d'un horaire et/ou d'une date, ou en fonction d'une concentration en composé gazeux (26).

9. Système de filtration (12) selon l'une quelconque des revendications 1 à 8, comprenant en outre un dispositif de protection des intempéries (80) comportant une membrane étanche à l'eau, perméable à l'air et translucide disposée sur la surface supérieure (40) de la première portion filtrante (34), ou comportant un matériau (82) translucide disposé à distance de la surface supérieure (40) de la première portion filtrante (34).

10. Système de filtration (12) selon l'une quelconque des revendications 1 à 9, dans lequel le matériau (35) ayant des propriétés catalytiques comprend une couche adsorbante (42) adaptée pour adsorber le composé gazeux (26), et une couche photocatalytique (44) comprenant au moins un élément photocatalytique (46) adapté pour dégrader et transformer le composé gazeux (26) adsorbé sur la couche adsorbante (42).

11. Système de filtration (12) selon la revendication 10, dans lequel l'élément photocatalytique (46) est l'oxyde de titane sous forme rutile.

12. Bassin (10) contenant un liquide (20) émettant un effluent gazeux pollué (24), l'effluent gazeux pollué (24) comprenant au moins un composé gazeux (26), ledit bassin (10) comprenant un système filtration (12) selon l'une quelconque des revendications 1 à 11, le dispositif de couverture (28) étant monté sur le bassin (10), la surface inférieure (38) de la première portion filtrante (34) étant orientée vers le liquide (20) du bassin (10), le dispositif de couverture (28), la surface du liquide (20) et au moins partie d'une paroi (16) du bassin (10) délimitant entre eux un espace de tête (32) contenant l'effluent gazeux pollué (24).

13. Bassin (10) selon la revendication 12, dans lequel au moins une partie de la première portion filtrante (34) s'étend dans un plan formant un angle non nul avec la surface du liquide (20).

14. Procédé de filtration d'un effluent gazeux pollué (24) émis par un liquide (20) contenu dans un bassin (10) selon la revendication 12 ou 13, ledit procédé comprenant les étapes suivantes :

   ◦ montage du dispositif de couverture (28) sur le bassin (10) avec les moyens de montage (30) de sorte à disposer la première portion filtrante (34) à distance du liquide (20),
   ◦ filtration de l'effluent gazeux pollué (24) par le passage dudit effluent (24) au travers de la première portion filtrante (34) de sorte à obtenir un effluent gazeux purifié (47).

15. Procédé de filtration selon la revendication 14, comprenant en outre une étape de contrôle d'une efficacité de filtration de la première portion filtrante (34), ladite étape comprenant les sous-étapes suivantes :

   ▪ mesure d'une première concentration en composé gazeux (26) de l'effluent gazeux pollué (24) dans l'espace de tête (32),
   ▪ mesure d'une deuxième concentration en composé gazeux (26) dans l'effluent gazeux purifié (47),
   ▪ calcul d'un paramètre représentatif de l'efficacité de filtration en fonction de la première concentration et de la deuxième concentration,
   ▪ remplacement ou nettoyage de la première portion filtrante (34) en fonction du paramètre représentatif de l'efficacité de filtration.

# Fig.1

# Fig.2

Fig.3

Fig.4

**Fig.5**

**Fig.6**

Europäisches
Patentamt

European
Patent Office

Office européen
des brevets

# RAPPORT DE RECHERCHE EUROPEENNE

Numéro de la demande

**EP 23 30 5420**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | CN 215 799 094 U (BEIJING HUAJIE ENVIRONMENTAL SCIENCE AND TECH CO LTD) 11 février 2022 (2022-02-11) | 1-4,9-15 | INV. B01D53/86 B01D53/88 |
| Y | * abrégé; figures 1,2 * | 5-8 | A01C3/02 A61L9/20 |
| | ----- | | B65D90/34 |
| Y | JP 2005 111321 A (SHOJI KENSETSU KK) 28 avril 2005 (2005-04-28) * alinéas [0019] – [0024]; figures 1-4 * | 5-8 | |
| | ----- | | |
| A | FR 2 938 447 A1 (ICARE [FR]) 21 mai 2010 (2010-05-21) * le document en entier * | 1-15 | |
| | ----- | | |
| A | KR 101 276 404 B1 (GEGLOBAL LNC [KR]; DONGYANG ENTECH CO LTD [KR] ET AL.) 19 juin 2013 (2013-06-19) * le document en entier * | 1-15 | |
| | ----- | | |

**DOMAINES TECHNIQUES RECHERCHES (IPC)**

B01D
B65D
A01C
A61L

Le présent rapport a été établi pour toutes les revendications

| Lieu de la recherche | Date d'achèvement de la recherche | Examinateur |
|---|---|---|
| La Haye | 15 septembre 2023 | Gruber, Marco |

EPO FORM 1503 03.82 (P04C02)

## ANNEXE AU RAPPORT DE RECHERCHE EUROPEENNE
## RELATIF A LA DEMANDE DE BREVET EUROPEEN NO.

**EP 23 30 5420**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**15-09-2023**

| Document brevet cité au rapport de recherche | Date de publication | Membre(s) de la famille de brevet(s) | Date de publication |
|---|---|---|---|
| CN 215799094 U | 11-02-2022 | AUCUN | |
| JP 2005111321 A | 28-04-2005 | JP 3903220 B2<br>JP 2005111321 A | 11-04-2007<br>28-04-2005 |
| FR 2938447 A1 | 21-05-2010 | AUCUN | |
| KR 101276404 B1 | 19-06-2013 | AUCUN | |

EPO FORM P0460

Pour tout renseignement concernant cette annexe : voir Journal Officiel de l'Office européen des brevets, No.12/82

## EP 4 438 160 A1